# EUROPEAN PATENT APPLICATION

(11) **EP 2 578 989 A1**
(43) Date of publication of application: **10.04.2013**
(21) Application number: 11184016.1
(22) Date of filing: 05.10.2011
(51) Int. Cl.: G01B 11/06

(54) **Distance sensor**

(71) Applicant: Optibake AB, 430 80 Asperö (SE)
(72) Inventor: Höhle, Martin, 42279 Wuppertal (DE); Rabus, Hans-Ulrich, 43080 Asperö (SE)
(74) Representative: Jönsson, Anneli

(57) **Abstract**

The invention relates a distance sensor 1 for monitoring proofing of dough comprising means associated with said distance sensor for producing an out-put signal when said distance sensor determines that a lump of dough 4 has achieved a predetermined size which is correlated with a desired degree of ripeness of the lump of dough. The distance sensor is a light interruption detector for detecting interruption of one or more light beams passing above at least one lump of dough and comprises a transmitter unit 2 and a receiver unit 3. The invention relates also to a device comprising the distance sensor as well as a method for monitoring proofing dough.

## Description

### Field of the invention

The invention relates to a distance sensor for detecting the proofing conditions of lumps of dough. The invention also relates to a device, comprising the distance sensor, for detecting the proofing conditions of lumps of dough, as well as a method for indicating the rising state of the lump of dough.

### Background of the invention

The typical bakery product such as bread or rolls, which are sold daily in large quantities by branches or retail outlets of bakery factories in order to be able to represent that they conform to the highest possible levels of freshness and quality, are baked in the respective branch or outlet locations, whereby it is also achieved that the produced amount can be flexible adapted to the respective demand.

In the production of table rolls the proofing process begins with lumps of dough, which are preproduced in a roll producing equipment in large number with substantially identical weight and volume. These lumps of dough, which are already subjected to yeast in their manufacture, are, prior to the rolls can be baked in an oven, subjected to a rising step in a proofing chamber, in which the size and dough concistency required for the baking of the rolls is strived for. Herein the proofing process is so carried out, with the respect to the chamber temperature and in certain cases the dwell time, that during a total proofing time of approximately 20 minutes the necessary dough size is achieved, which in typical cases represents the five fold of the roll producing devices before the initiation of the rapidly occurring proofing process is set in motion therein by the elevation of the temperature, after the course of which, that is as soon as the dough lumps have achieved their desired size, these should be baked immediately, in order to achieve table rolls of standardized desired quality. The most important characteristic of quality of the finished baked rolls, which is associated in the mind of consumer with the image of quality, is the size thereof, which should also be uniform among the various types of rolls.

In order to achieve the uniformity, it would seem to be basically suitable to employ constant uniform conditions for carrying out the proofing process, for example by combining a computer program controlled time-wise control of the proofing chamber temperature with an indication that the proofing time has concluded as determined by the temperature profile.

However, only control of temperature during the proofing process is not enough for securing or improving the quality of the product.

The proofing or fermenting process of the bakery products may, for example be induced by yeast, in course of which the lumps of dough experience an increase in volume. Parameters like time and temperature when monitoring the proofing of dough have been used for indicating but are not reliable enough for optimizing the proofing process. The proofing process may also be controlled by a visual quality control of the lump doughs. The demand of a high level of practical experience, in particular in that respect of how in certain cases a proofing process, is to be carried out to a suitable ripeness of the dough lumps.

Another parameters affecting the result are the condition of the yeast added in the producing the dough, the older yeast the longer time period is required for proofing the yeast. Further, the proofing is affected by the humidity, the temperature, the salt content of the dough, the water content, the content of starch of the meal added etc. However, neither these parameters are reliable enough for determining when the proofing of the dough is optimal.

By measuring or monitoring the volume of the dough the proofing process may be optimized. The monitoring of volume changes may be achieved by monitoring the changes of height of the lump dough.

During the proofing, the sensible properties like taste and texture are developed. A dough not enough proofed is flavorless in comparison with an optimal fermented dough.

Also, during the proofing the nutrition value of the dough and of the final bakery product is developed. The amount of vitamins in the dough is increasing until an optimum is reached during the proofing. If the proofing is shortened, the nutrition value may be lower than it would be possible to obtain if the proofing process had been optimized, as a less amount vitamins is allowed to be developed in the dough.

Also the visible appearance of the dough and the final pastry is less esthetically favorable.

Thus, the proofing process of the dough is important for the final result of the pastry, both when considering the nutrition value of the final pastry, the taste sensation of the pastry, as well as the estethical appearance of the pastry product. Most often it is only after the bakery product has been baked that it can be recognized that the different quality requirements are fulfilled.

The above applies for all different forms and types of bakery products, such as bread, rolls etc.

From US 6 397 734 a device for detecting the rising state of the lumps of dough. The device for detecting includes a distance sensor operating by a light interruption detector, thus distance sensor indicates when the light beam is interrupted by a rising lump of dough. However, the method is not reliable enough, for example as only one light beam is used to indicate the status of the proofing process.

There is a need for detecting devices and methods which more reliable indicates the optimal rising height of the lumps of dough, than the previously known devices and methods.

The present invention provides a device which enables to monitorise the proofing process.

### Summary of the invention

The present invention provides a distance sensor for detecting the rising of the lumps of dough wherein the distance sensor indicates the optimal proofing by monitoring the rising height, which is correlated to the desired final size and quality of the bakery product final.

The distance sensor indicates when the proofing of lump dough has reached a predetermined optimal size.

In one embodiment of the invention a distance sensor for monitoring proofing of dough, comprising means associated with said distance sensor for producing an out-put signal when said distance sensor determines that a lump of dough has achieved a predetermined intended thickness value, which is correlated with a desired degree of ripeness of the lump of dough is provided. The distance sensor is a light interruption detector for detecting interruption of one or more substantial horizontal light beams passing above at least one lump of dough; and comprises a transmitter unit having one or more optical emitters emitting the said substantial horizontal light beam, and a receiver unit comprising one or more optical receivers, receiving the light beams.

An advantage with the distance sensor of the invention is a more reliable detection of the proofing process is available. By that the quality of the lumps of dough being subjected for proofing may be improved when considering the taste, texture and estethically appearance. Therefore the amount of bakery products which can be exposed to the customers are considerable higher than when previously known devices are used.

The distance sensor of the invention only requires a small amount of space and can be installed between two supporting surfaces, such as proofing sheets, for dough lumps within a proofing chamber.

In another embodiment of the invention the distance sensor for detecting is arranged to cross over multiple lumps of dough. An example, a centrally oriented row of lumps of dough, whereby even in case of minor irregular arrangement of the lumps of dough the probability is increased that the light detector is interrupted at the correct point in time, even when the light beam is interrupted at the correct point in time, even when the light detector-light beam does not precisely cross over the central plane of some of the individual dough lumps.

In one embodiment of the invention a distance sensor for monitoring proofing of dough as defined above is provided, wherein the light beams are emitted from the first side of the lump of dough; passing substantially above the central part of the lump of dough; and received at the second side of the lump of dough.

In the distance sensor the light emitting may also be performed by substantial horizontal arranged parallel light beams.

The distance sensor according to the invention comprises a number of optical emitters which differs from the number of optical receivers.

Preferably the number of optical emitters is at least X+1, wherein X denotes the number optical receivers. Another example is wherein the ratio of optical receivers to optical emitters is between 1:2 and 1:15; preferably between 1:2 and 1:10; preferably between 1:2 and 1:5.

In one embodiment of the distance sensor, the optical receivers are mounted in vertical rows and parallel arranged to each other.

Further, the distance sensor as above may also monitor other features than the change of distance of the dough. For example features like temperature and humidity present in the space around the lump of dough may be measured and monitored. Thus the distance sensor may also comprise means for measuring temperature of the proofing chamber, as well as means for measuring the humidity of the proofing chamber.

These means for monitories the proofing process may provide the possibility to recapitulate the proofing process and baking process if so needed. The quality assurance may be improved if the process details may be monitorised, and also, are available for back-logging.

In an embodiment of the invention, a device for detecting the proofing conditions of lumps of dough during a proofing process in a proofing chamber, in the course of which the lumps of dough experience an increase in volume, comprising the distance sensor as defined above, is provided.The distance sensor may be arranged in the device for detecting the state of rising the lumps of dough. The said device may be used for indicating the rising state of one lump of dough as well as multiple lumps of doughs. The device comprises the distance sensor which may be placed on a proofing sheet upon multiple lumps of dough are placed.

In another embodiment of the invention a method for monitoring the proofing of dough comprising detecting the proofing conditions of lumps of dough in a proofing process in the course of which the lumps of dough experience an increase in volume, comprising the following steps:
- placing the lumps of dough subjecting for proofing on a supporting sheet;
- monitoring of light beams emitted from the first side of the lump of dough; received on the second side of the lump of dough;
- producing an out-put signal when the monitoring of light beams determines that a lump of dough has achieved a predetermined intended thickness value, which is correlated with a desired degree of ripeness of the lump of dough caused by an interruption of one or more light beams passing above the central area of at least one lump of dough in the proofing chamber, and is detected by a transmitter unit having optical emitters and a receiver unit having optical receivers.

The number of said optical receivers used for the method may be less than the number of optical emitters.

In the method the light emitting is performed by horizontal arranged parallel light beams. Also, the method may include monitoring the temperature at which the proofing process, as well as monitoring the humidity of the air at which the proofing process is performed.

The distance sensor, the device and the method provides means for an objective determination of the proofing process and the condition of ripeness of the monitored dough lump. The distance sensor and the device open up for, depending upon the instantaneously detected volume of the dough lumps, to control the proofing process by influencing the proofing chamber temperature in such a manner, so that the condition of ripeness necessary for the subsequent baking process of the dough lumps is achieved after the expiration of a pre-determined time span. Further, the process is easier to repeat than with previous known methods. A more efficient and rational baking procedure may be achieved by the present invention.

### Brief description of the figures

Figure 1 illustrates a device for detecting proofing conditions of lumps of doug.
Figure 2 illustrates the distance sensor, implemented in the device of the invention, shown as side view as (figure 2a) and as a top view (figure 2b).
Figure 3 illustrate schematically the arrangement of transmitter unit with optical emitters and of receiver unit with optical receivers, shown as side view (figure 3a), and as top view (figure 3b).
Figure 4 illustrates examples of printing boards, in figure 4a the emitting unit, in figure 4b the receiving unit.

### Detailed description of the invention

The device 15 is for detecting the proofing conditions of lumps of dough during a proofing process. The device comprises a proofing cabinet 31, a proofing chamber 30, and, in the course of which the lumps of dough 4 experience an increase in volume, and the distance sensor 1 of the invention.

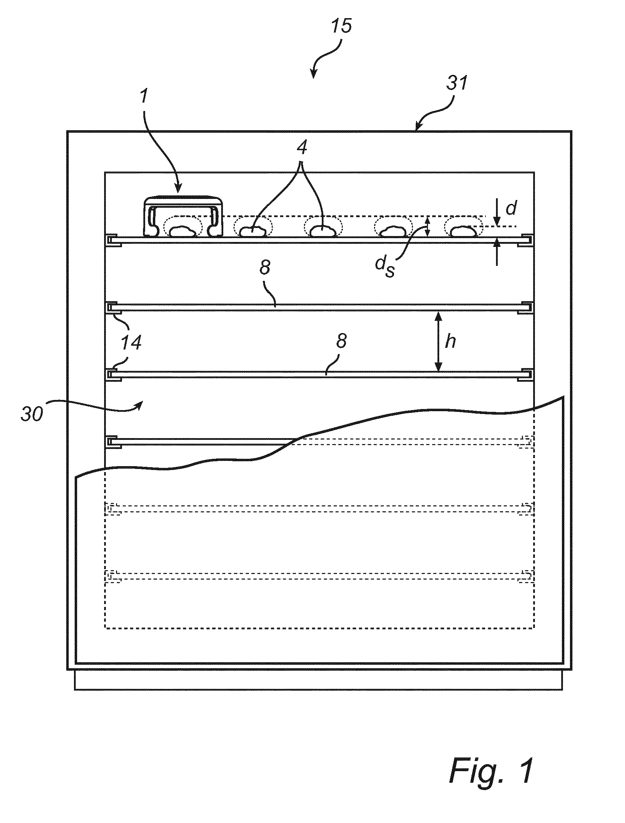

The distance sensor 1 may be arranged therein for detecting the state of rising the lumps of dough. The said device may be flexible in size, it may be used for indicating the rising state of one lump of dough as well as multiple lumps of doughs. In figure 1, the device is suitable for multiple lumps of doughs, placed on several proofing sheets.

In the proofing chamber 30, lumps of dough 4, for example table rolls or bread, which can be subjected to a proofing process, before they can be finally baked in a baking oven (not shown) immediately after completion of the proofing process.

By means of this proofing process the lumps of dough which undergo a substantial increase in volume during the proofing process, are to be brought to the suitable consistency of the dough for baking, which brings about once again further volume increase up to the final size of the finished rolls.

Herein the lumps of dough are placed on one or more proofing sheets 8which are easily moved in the sideways guide rails 14, which facilitates the easy introduction of the dough lumps into the proofing chamber 30 of the proofing cabinet and the removal thereof. These proofing sheets 8 which have an approximately square carrying surface 9 are provided respectively in the same spatial separation h from each other, which for the purpose of explanation will be presumed herein to be approximately 70 mm, wherein it is presumed that the intended thickness ds of the lumps of dough 4 as a result of the proofing process, is to have a vertical final thickness of approximately 50 mm. In the design presumed for the proofing cabinet 30 used in this explanation, 6 proofing sheets 12 are provided, on which batches of respectively 25 lumps of dough can be deposited.

The proofing cabinet 31 is provided with hot air convection heating device (not shown), by means of which the temperature produced in the proofing chamber 30 is adjustable and can be changed or varied to conform to a product-optimal temperature curve, over which the proofing process is controllable, wherein various temperature can be pre-selected and be run by control programs.

For monitoring the proofing process, which results in an increase in volume of the lumps of dough, a distance sensor 1 indicated overall with s provided, by means of which the vertical thickness d of a selected lump of dough 4 is determinable, which is monitored as criteria for the degree of ripeness of the lumps of dough 4 subjected to the proofing process. As soon as the lumps of dough have achieved the pre-determined intended thickness (ds), and the light beams at this height are interrupted, the distance sensor 1 produces an electric output signal characteristic therefore, which serves as an indicator signal signifying that the lumps of dough have achieved their condition of readiness for baking.

An option is also that there is a signal for controlling and for reducing the temperature in the proofing chamber 30, in order to stop the proofing process.

Also, the invention provides a method for monitoring the proofing of dough. By the method, the proofing conditions of lumps of dough are detected during the proofing process in the course of which the lumps of dough experience an increase in volume. The method comprises the steps like: placing the lumps of dough subjecting for proofing on a supporting sheet. Depending on the size of the device 15, and the number of proofing sheet, the lump of doughs to be placed may be one or more. The proofing of the lump of doughs is then followed by: monitoring of light beams emitted from the first side of the lump of dough; received on the second side of the lump of dough. The transmitter unit 2 and the receiver unit 3 of the distance sensor are placed in a way that substantial horizontal light beams are emitted over, central part of the lump of dough.

When the dough has achieved a predetermined intended thickness value ds, which has been correlated with a desired degree of ripeness of the lump of dough the proofing process an out-put signal is produced by. The signal is indicated by an interruption of one or more light beams passing above the central area of at least one lump of dough in the proofing chamber, and is detected by a transmitter unit having optical emitters and a receiver unit having optical receivers.

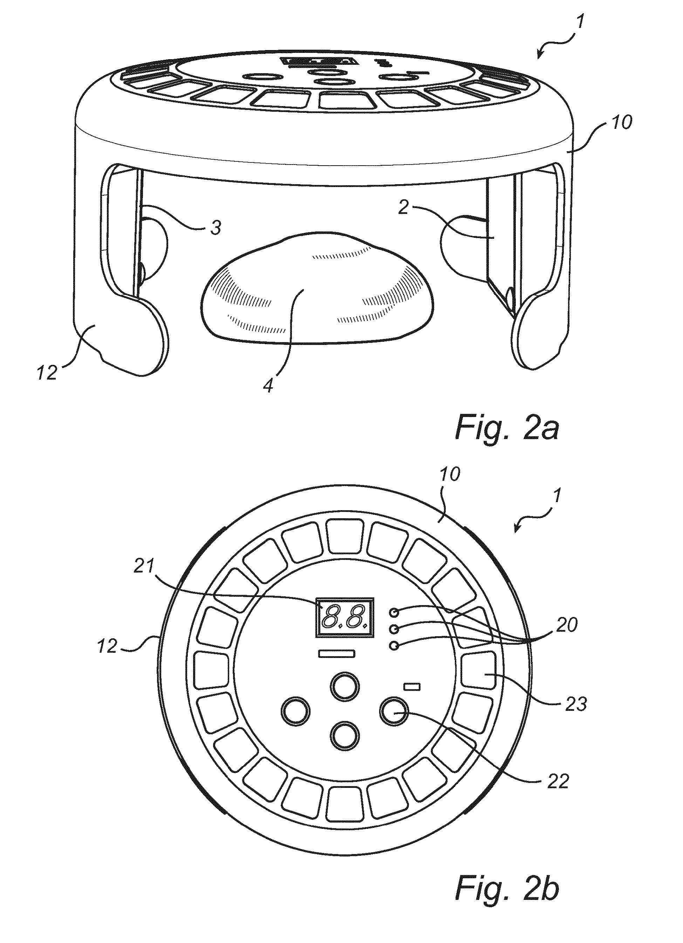

The distance sensor 1 is now to be explained in greater detail by reference to figure 2a and figure 2b.

The distance sensor 1 comprising a carrying framework 10 in the manner of a "table top" being legged in a way to make it suitable to place above the lumps of dough being subjected to the proofing process is shown in figure 2a. For example the carrying framework 10 is provided with two, or more, supporting legs 12. It is important that the environment around the lump dough placed under the distance sensor is substantially the same as the environment around the dough lumps 4 placed on the proofing sheet but not being subject for the measurement by a distance sensor. Therefore, there are no limits of the design and arrangement of the carrying framework as long as distance sensor may operate for optimizing the proofing process.

A transmitter unit 3 is arranged so that it emits a light beam from one side, the first side, of the lump of dough. The transmitter unit 2, comprising one or more optical emitters 25, is mounted on one of the supporting legs 12.

The receiver unit is arranged to be placed on the other, second, part of the dough. The receiver unit 3, comprising one or more optical receivers 35, is mounted on a supporting leg placed on the other side of, for example the opposite to, the optical emitters. In an arrangement, multiple receivers are arranged together into a "multiple receiver".

The arrangement of the transmitter unit 2 and the receiving unit 3 accepts all possible arrangements of the equipment, as long as the transmitter unit is placed on one side, the first side, of the dough, and the receiving unit is placed on the other side, the second side, of the dough, for example, they are arranged substantially opposite to each other.

The optical emitters may be any suitable light emitting source. They may be mounted separately or as modules comprising multiple optical emitters (a so-called sensor-module).

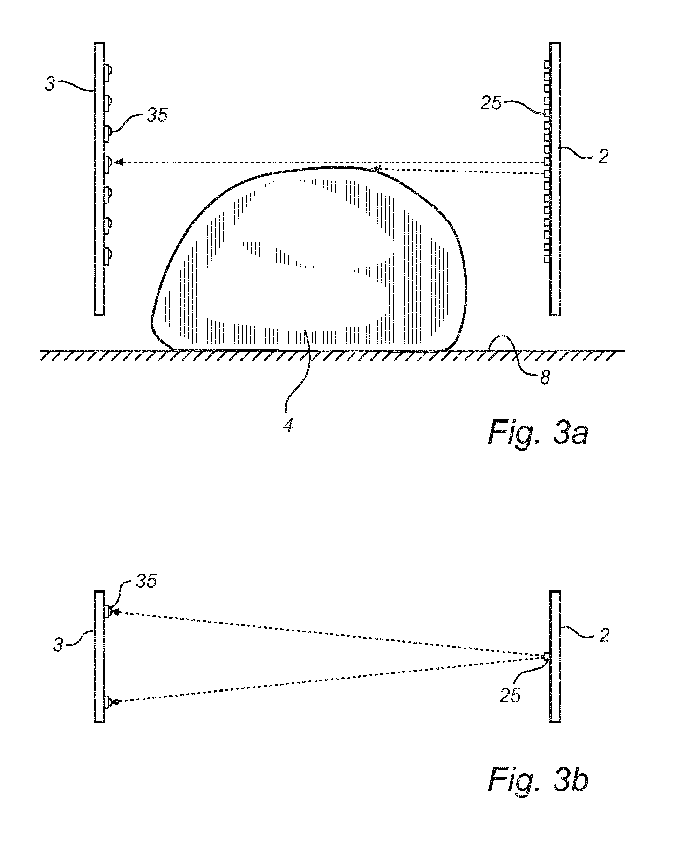

The optical emitters are to be arranged in a manner generating horizontal light beams to be received by the receiving unit. This is further shown in Figure 3.

The transmitter unit 2 comprising the optical emitters 25 emit the light beam horizontal, preferably substantially parallel to the contradictory receiver unit 3, mounting one or more optical receivers 35. This arrangement broadens the application of the distance sensor to be used for many different sizes of the lump of doughs.

The lump of dough 4 placed on the proofing sheet is let to proof up to a predetermined size, corresponding to the thickness ds. When the lump of dough interrupts a light beam, at the predetermined height corresponding to just that type of dough lump, a signal indicates that the proofing process is finalized.

The out-put signal may be of any kind or combinations of several, for example visible signal, audible signal.

The arrangement of the transmitter unit 2 and the receiving unit 3 accepts all possible arrangements of the equipment for considering the light scattering in an optimal way. According to the invention, the light beams are `substantially horizontal' when related to the proofing sheet. Most all optical emitters available scatter the light beams in some way. The scattering of light beam is a parameter to consider when selecting the optical emitters, and the following arrangement. Preferably, the optical emitters selected for the present invention show scattering less than 10 degrees, preferably less than 7 degrees, when arranged in the distance sensor of the invention. The scattering of the light beam is also depending on the length of the beam. Further, particles present in the air in the proofing chamber affect the light scattering. For example water vapor present in the proofing chamber may affect the light scattering and by that the accuracy and resolution of the measurement. These parameters are acceptable and included in the definition of the `substantially horizontal' light beam.

The resolution of the light beam may be affected by the number of emitters included in the transmitter unit 2. Another factor to consider is position of the emitters used in the range.

The accuracy of the light emitting may be affected by optical size of the light source. The receiving unit 3 is so designed and positioned that it preferably receives the light beams substantial in parallel.

The present invention provides a device for detecting the proofing condition of lumps of dough during a proofing process.

The height of the measurement is dependent on the range of light emitters. The range of the light emitters may be dependent on the purpose of the distance sensor, thus the kind of bakery product. The duration of the measurement may depend on the power of the light source used in the distance sensor, as well as the algoritm used for the measurement.

The measurement may take place during a predetermined time period. The device may be optimized in this respect regarding power consumption and life time of the product and its components. An example of the measurement, a single measurement of the proofing dough may take place during a time period of 2 to 10 seconds.

The result is treated and calculated, the result transmitted further is a mean value of the single measurement. This procedure, or 'measurement cycle', is repeated frequently, with a predetermined frequency. For example, a measurement cycle is performed every 20 second.

One or more additional light beams arranged in parallel may be used, which also instantly require additional optical receivers, for example, one more per each additional light beam.

The accuracy of the result obtained in increase by using several light beams. However, this increased accuracy is connected with increased cost for the equipment, which may be a parameter to consider.

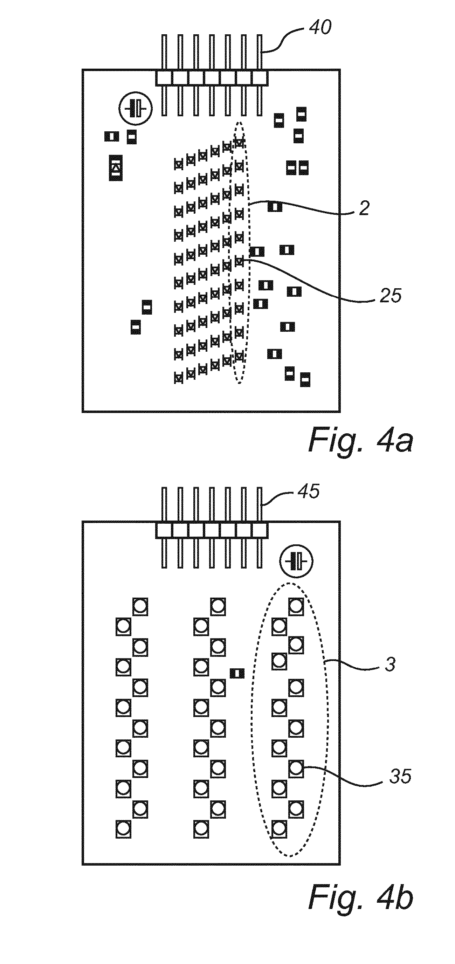

In figure 4 an example of a transmitter unit (figure 4a) and an example of corresponding receiving unit (figure 4b) arranged upon printing boards are illustrated.

From figure 4 the displacements of the optical emitters, and the receivers are shown as printing boards including the connecting means 40, 45. The arrangement of the emitters increases the resolution of the light beam. The arrangement provides several light beams to be sent to the receiever unit.

The receivers, herein shown as three vertical rows, where each row comprises a couple of receivers, lateral displaced to each other. They may be correlated to the light scattering of the emitters.

The system may also be calibrated regularly, where the positions and values are adjusted and corrected.

In one example of the invention the communication between the distance sensor and the transmitter device connected thereto is via serial line. A controlling system is connected to the device for detecting the proofing condition, the connection to this superordinated controlling system is via the asynchrony serial interface on the transmitter unit 2. A communication protocol is implemented, which may be based on 7 bit ASCII. According to the example the communication is via single letter commands coded in ASCII.

When using the distance sensor in normal measurement mode, the device transmits the last result value as an ASCII string every second. The value is the actual height expressed in 1/11 mm followed by a carriage return (defined as 0x0d) and the feed (defined as 0x0a) character. According to the example the result was in the range of 0 to 3810.

More thoroughly explained:
A lump of dough having a heigt of 5.71 mm was communicated like:

| String | ASCII | Codes on line (hex) |
|---|---|---|
| 571 | '5' '7' '1' 'cr' 'If' | 35 b7 b1 8d 0a |

When preparing for calibration, the command 'k' was sent. The measurement mode is interrupted transmitting a cr/If sequence. The distance sensor is then changed into calibration mode.

According to the example the height to be measured is in the range of 38 mm comprising 60 emitters.

The resolution is 0.635 mm; and the accuracy obtainable is two times the resolution. A measurement may be reproduced by 0.635 mm.

The present invention provides a method to monitorise the proofing process by a broader area of light beams than what is achieved by only a single light source, and by that a single light beam. Depending on the surface of the lump of dough the measurement and monitorising of the proofing process can be unreliable and unpredictable. The surface may be more or less rough depending on the type of bakery product and the meal included. Also, during the proofing process small bumps and bubbles may be formed which affect the monitorising of the proofing process. The present invention provides means where the measurement and monitorising are more stable and reliable as a broader area of light beams to be interrupted by the proofing dough is provided, thus, not only a single light beam.

Having now fully described this invention it will be appreciated by those skilled in the art that the same can be performed within a wide range of equivalent parameters. The variations to the disclosed embodiments can be understood and effected by those skilled in the art practising the claimed invention, from studying the disclosure and appended claims, and without departing from the gist and scope of the invention and without undue experimentation. This application is intended to cover any variations, uses, or adaptations of the invention following, in general, the principles of the invention and including such departures from the present disclusure as come within known or customary practice within the art to which the invention pertains and as may be applied to.

## Claims

1. A distance sensor 1 for monitoring proofing of dough comprising means associated with said distance sensor for producing an out-put signal when said distance sensor determines that a lump of dough 4 has achieved a predetermined intended thickness value, which is correlated with a desired degree of ripeness of the lump of dough; wherein the distance sensor 1 is a light interruption detector for detecting interruption of one or more light beams passing above at least one lump of dough, comprising a transmitter unit 2 comprising one or more optical emitters 25, and a receiver unit 3 comprising one or more optical receivers 35.

2. A distance sensor 1 for monitoring proofing of dough according to claim 1 wherein the light beams are emitted from one side of the lump of dough 4; passing substantially above the central part of the lump of dough; and received by receiving unit placed at the other side of the lump of dough.

3. The distance sensor according to claim 1 or claim 2, wherein the light beams are emitted as substantially horizontal light beams.

4. The distance sensor according to any of preceding claims 1 to 3, wherein the number of optical emitters 25 differs from the number of optical receivers; preferably the number of optical emitters is at least X+1, wherein X denotes the number optical receivers.

5. The distance sensor according to any of claim 1 to 4, wherein the ratio of optical receivers 35 to optical emitters is between 1:2 and 1:15; preferably between 1:2 and 1:10; preferably between 1:2 and 1:5.

6. The distance sensor according to any of claims 1 wherein the optical emitters 25 are mounted in vertical rows, and the optical receivers 35 are mounted in vertical rows.

7. The distance sensor according to claim 1 wherein said device comprises means for measuring temperature of the proofing chamber 30.

8. The distance sensor according to claim 1 wherein said device comprises means for measuring the humidity of the proofing chamber 30.

9. A device for detecting the proofing conditions of lumps of dough during a proofing process in a proofing chamber, in the course of which the lumps of dough experience an increase in volume, comprising a distance sensor 1 as defined in claims 1 to 8.

10. Method for monitoring the proofing of dough comprising detecting the proofing conditions of lumps of dough in a proofing process in the course of which the lumps of dough experience an increase in volume, comprising the following steps:
- placing the lumps of dough 4 subjecting for proofing on a proofing sheet 8;
- monitoring of light beams emitted from the one side of the lump of dough; received on the other side of the lump of dough;
- producing an out-put signal when the monitoring of light beams determines that a lump of dough has achieved a predetermined intended thickness value, which is correlated with a desired degree of ripeness of the lump of dough caused by an interruption of one or more light beams passing above the central area of at least one lump of dough in the proofing chamber, from and is detected by a transmitter unit having optical emitters and a receiver unit having optical receivers.

11. The method according to claim 10 wherein the number of said optical receivers 35 is in less than the number of optical emitters 25 are less than the number of said optical receivers.

12. The method according to any of claims 10 to 11 wherein light emitting is performed by substantially horizontal arranged light beams.

13. The method according to claim 10 wherein the transmitter unit transmits the result as an ASCII string with predetermined time intervals.

14. The method according to claim 10 including monitoring the temperature at which the proofing process is performed.

15. The method according to claim X including monitoring the humidity of the air at which the proofing process is performed.
